Europäisches Patentamt

**European Patent Office**

Office européen des brevets

(11) Publication number: **0 108 529**
**A1**

(12) # EUROPEAN PATENT APPLICATION

(21) Application number: **83306317.5**

(22) Date of filing: **18.10.83**

(51) Int. Cl.³: **A 61 M 5/20**

(30) Priority: **20.10.82 ZA 827678**

(43) Date of publication of application:
**16.05.84 Bulletin 84/20**

(84) Designated Contracting States:
**AT BE CH DE FR GB IT LI LU NL SE**

(71) Applicant: **PEIGNOIR INVESTMENTS (PROPRIETARY) LIMITED**
**50 Van Riebeeck Avenue**
**Alberton Transvaal Province(ZA)**

(72) Inventor: **Pienaar, Daniel Petrus**
**117 Bellefield Avenue**
**Mondeor Johannesburg Transvaal Provincie(ZA)**

(74) Representative: **Dixon, Donald Cossar et al,**
**Gee & Co. Chancery House Chancery Lane**
**London WC2A 1QU(GB)**

(54) A device for operating a syringe.

(57) A device (10) for operating a syringe includes a first support member (12) having formations (54, 56) to locate a syringe with respect thereto and a second support member (16, 17) onto which the first support member is displaceably mounted. An operating member (64) is displaceably mounted onto the first support member (12) and in combination the above members are displaceable with respect to one another utilising suitable holding means (28, 38) between a "cocked" position in which the syringe located on the first support member (12) and its plunger is retracted and a "triggered" position in which the needle of the syringe has first been inserted into the body of a person and thereafter the plunger has been displaced to inject a liquid from the syringe into the body of the person by the displacement of the first support member (12) with respect to the second support member (16, 17) and the operating member (64) with respect to the first support member (12) respectively.

FIG. 2

EP 0 108 529 A1

## A DEVICE FOR OPERATING A SYRINGE

THIS INVENTION relates to a device for operating a syringe to inject fluids into the body of a person.

A syringe conventionally includes a tubular container or cylinder within which a plunger is displaceable to eject a fluid from the container, via a suitable needle, into the body of a person. Displacement of the plunger is usually manually effected by displacing a plunger rod which projects from the container and is secured to the plunger.

According to the invention there is provided a device for operating a syringe to inject fluids into the body of a person, which includes

a first support member including locating formations for removably locating and supporting a syringe;

a second support member onto which the first support member is displaceably mounted for displacement with respect to the second support member from a first position into a second position during which the needle of a syringe, supported by the first support member, can be inserted into the body of a person while the second support member is held in a predetermined steady relationship with respect to the body of the person;

an operating member displaceably mounted onto the first support member for displacement with respect to the first support member from a first position into a second position during which it can engage and displace the plunger of a syringe, supported by the first support member, for injecting a fluid from the syringe into the body of a person;

urging means for urging the first support member and the operating member into their second positions;

a first holding mechanism for releasably holding the first support member in its first position, with respect to the second support member, being operable to release the first support member in order to permit its displacement into its second position;    and

a second holding mechanism for releasably holding the operating member in its first position with respect to the first support member being operable to release the operating member in order to permit its displacement into its second position only when the first support member is in its second position with respect to the second support member.

The first support member may be linearly displaceable with respect to the second support member between its first position in which the needle of a syringe supported by the first support member in use, is effectively retracted, and its second position in which the needle is inserted into the body of a person.

The urging means may include two springs which can act respectively on the first support member and the operating member for urging them into their second positions.

Preferably, the first holding mechanism may be manually operable to release the first support member in order to permit its displacement into its second position. In particular, the first holding mechanism may include a hook formation secured to the second support member and urged into engagement with an engagement formation of the first support member when in its first position, being manually displaceable to release the engagement formation and permit displacement of the first support member into its second position.

Further, according to the invention, the operating member may be linearly displaceable with respect to the first support member between its first position in which the plunger of a syringe supported by the first support member is effectively retracted and its second position in which a fluid contained in the container of the syringe has been ejected by the displacement of the plunger.

Conveniently, operation of the second holding mechanism may be sensitive to the displacement of the first support member and may be actuated to release the operating member by means associated with the first support member to permit displacement of the operating member.

Furthermore, the second holding mechanism may be adapted to hold the operating member with respect to the first support member in a plurality of positions which determine the first position thereof. More particularly, the second holding mechanism may be a pawl and ratchet-type mechanism, ratchet formations being defined by the operating member which are engageable by a pawl formation urged into engagement with the ratchet formations to hold the operating member in a required position, the pawl formation being displaceable in response to displacement of the first support member into its second position to release the operating member and permit its displacement into its second position.

In addition, the device of the invention may include adjustment means for adjusting the distance of insertion of a needle into the body of a person. The adjustment means may be an abutment formation, adjustably securable to the second support member, which abuts the body of a person, in use, and which determines the position of a syringe, when supported by the first support member, with respect to the body of the person and thus the distance of insertion into the body of the person.

The adjustment means may further include a pawl and ratchet-type mechanism which permits adjustment of the position of the abutment formation with respect to the second support member.

Preferably, the locating formations of the first support member may be such that they can locate syringes having a range of shapes and sizes.

In a particular configuration of the invention the operating member may define a piston and the first support member a cylinder within which the piston is linearly displaceable, to provide for the displacement of the operating member. For this configuration, a fluid outlet means may be defined to release fluid from the cylinder at a controlled rate to thereby control the rate of displacement of the operating member. The fluid outlet means may also be in communication with a liquid container defining a closed liquid flow path with the container and the cylinder.

The device may further include a cover member mounted onto the second support member being displaceable to permit or inhibit access to a syringe located on the first support member, in use.

The device of the invention can clearly be provided in various constructional modes. In particular, each component of the device can be provided in various forms whilst still falling within the broad scope of the present invention.

The operating device of the invention may be particularly suitable for sub-cutaneous injection in which case a fluid is merely injected into the body of a person beneath the skin. More particularly, the operating device of the invention is particularly suitable for the injection of insulin into the body of a person for the treatment of diabetes.

The invention is now described, by way of example, with reference to the accompanying diagrammatic drawings, in which

Figure 1 shows a plan view of a device for operating a syringe in accordance with the invention;

Figure 2 shows a sectional side view of the device of Figure 1 along line II-II of Figure 1;

Figure 3 shows a sectional end view of the device of Figure 1 along line III-III of Figure 2; and

Figure 4 shows a sectional end view of the device of Figure 1 along IV-IV of Figure 2.

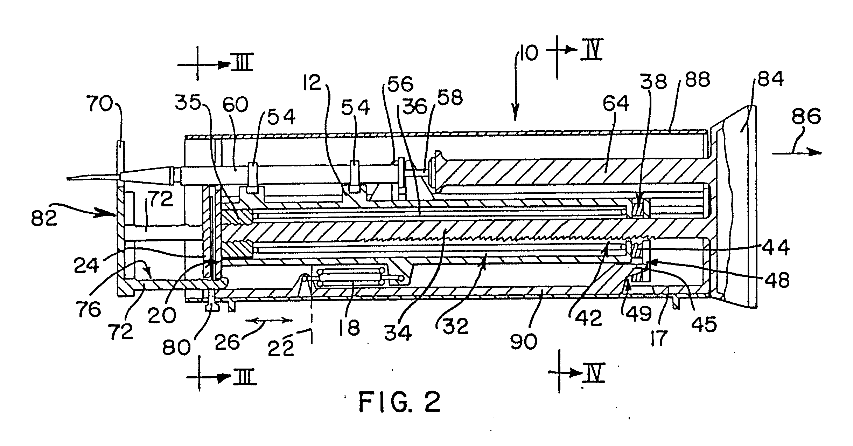

Referring to the drawings, a device for operating a syringe to inject a fluid into the body of a person, in accordance with the invention, is generally indicated by the reference numeral 10. The device 10 includes a first support member 12 which engages opposite elongate slots 14 defined along the length of a second support member including two guide elements 16, so that the member 12 is linearly and slidably displaceable within the slots 14 with respect to the guide elements 16. The guide elements 16 are secured to a base member 17 which then also forms a part of the second support member. In its operative configuration within the slots 14, the first support member 12 is engaged with the base member 17 of the second support member by means of a tension spring 18 which is tensioned when the first support member 12 is retracted into a first position where its front end 20 is aligned with dotted line 22 (shown in Figure 2). It will be appreciated that when in this retracted position the first support member 12 will be urged, by means of the spring 18 towards its second position in which its front end 20 abuts the front wall 24 of the base member 17 forming part of the second support member. The front wall 24 thus defines the second position of the member 12 with respect to the second support member. The relative displacement between the first and second support members is therefore indicated by arrows 26.

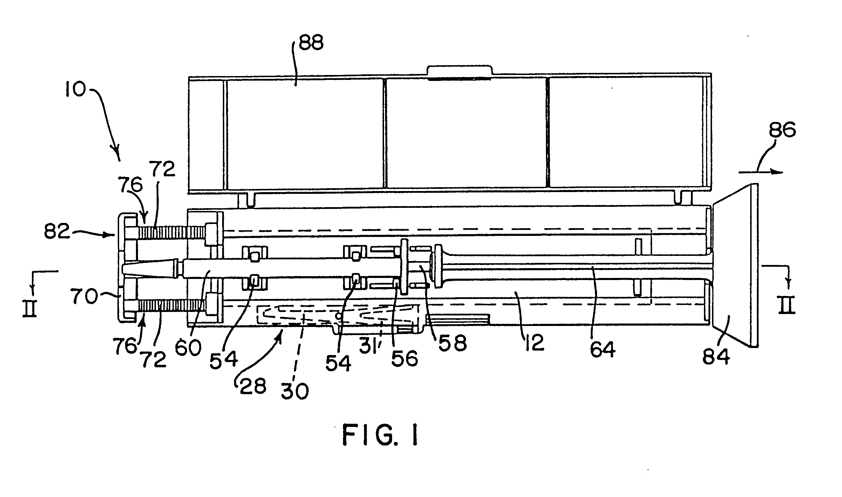

A first holding mechanism generally indicated by the reference numeral 28 is provided to hold the first support member 12 in its retracted position with respect to the second support member. The exact operation of the holding mechanism 28 is not described in detail herein but essentially provides for a manually displaceable hook formation 30 which is urged into engagement with a complementary formation (not shown) defined by the first support member 12 so that when the first support member 16 is retracted it is engaged by the hook formation 30. Also, by manual depression of the arm 31 the first support member 12 is released so that it is displaced by the urging force of the spring 18 towards the position as is described hereinabove. The specific construction of the hook formation as shown clearly in Figure 1 provides for the required operation thereof.

The first support member 12 further defines an elongate chamber 32 within which a rod 34 is axially displaceable along a line parallel to the line of displacement of the first support member 12 with respect to the second support member. The rod is guided by a plug 35 secured at its free end and is urged towards the end 20 of the first support member 16 by means of a tension spring 36. The rod 34 can be held in a retracted position by means of a pawl and ratchet.arrangement generally indicated by the reference numeral 38. In particular, the rod 34 has ratchet formations 42 defined along one of its sides and a pawl 44 is urged, by means of a compression spring or the like (not shown) into engagement with the ratchet formations 42 for holding the rod 34 in a retracted positon. An aperture 48 through the pawl 44 is partially aligned with a lug 45 forming part of and projecting from the base member 17 forming part of the second support member. From Figure 2 of the drawings, it will be clear that when the first support member 12 is in its retracted position with respect to the second support member, the pawl 44 will be in engagement with the ratchet formations 42 and can thus hold the rod 34 in a retracted position. However, when the first support member 12 is displaced into its second position as shown in the drawing, the lug 45 will pass through the aperture 48 with its sloping operative edge 49 engaging the operative remote surface of the aperture 48 with respect to the ratchet formations 42. As a result, the pawl 44 is pushed downwardly due to this engagement, against its bias, providing for disengagement with the ratchet formations 42 and permitting displacement of the rod 34 towards the end 20 of the first support member 12.

The top portion of the first support member 12 above the chamber 32 defines receiving formations 54 adapted to receive a conventional type syringe. Such a syringe will lie within the formations 44 and usually defines shoulders which can engage location formations 56. The formations 54 and 56 can clearly be adapted to receive any particular type syringe or a range of types of syringes.

When positioned within the formations 54 and 56, the piston rod 58 of a syringe 60 will project towards an operating member 64 which is integral with the rod 14 displaceable in the chamber 32. It will be clear from Figures 1 and 2 of the drawings that by displacement of the operating member 64, the piston rod of a syringe can be displaced with respect to the remainder of the syringe causing the ejection of fluids from the syringe. The required stroke of the piston rod of the syringe clearly determines the required distance of displacement of the operating member 64 which in turn is determined by the position in which it is held with respect to the first support member 12 by means of the pawl and ratchet arrangement 38.

If required, to control the rate of displacement of the rod 34 within the chamber 32, the chamber may be blocked off and the plug 35 may be dimensioned such that it sealingly engages the walls of the chamber 32 forming an effective piston.  An air release valve (not shown) may then be located near the end 20 of the support member 12, this valve permitting release of air from the chamber 32 via a passage (not shown) leading from the chamber.  It will be appreciated that by controlling the rate of air release, the rate of displacement of the piston within the chamber 32 can also be suitably controlled.  Alternative arrangements for this purpose is also anticipated by the Applicant utilising, for example, the displacement of a liquid from the chamber 32.

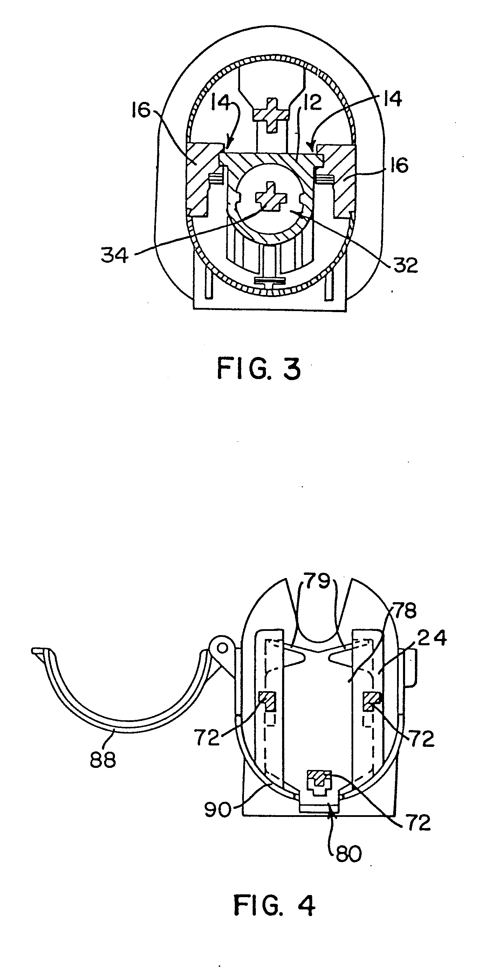

A displaceable abutment formation 70 has three guide elements 72 projecting therefrom, the elements 72 passing through apertures (not shown clearly) defined in the front wall 24 of the base member 17.  Each element 72 defines ratchet formations 76 along one edge thereof which are engageable by an engagement plate 78, displaceably housed within the front wall 24, defining complementary formations, in a pawl and ratchet fashion.  The plate 78 is urged into engagement with the elements 72 by resilient formations 79 as shown in Figure 4 and is manually displaceable to adjust the position of the abutment formation 70 with respect to the first support member 12 by depressing the push-button 80 engaged with the plate 78 and projecting from the device 10 as shown. By such adjustment, the effective distance of insertion of the needle of a syringe into the body of a person can clearly be adjusted since the formation 70 is displaceable with respect to the syringe and is utilised to abut the body of a person in use, to thereby enable a person to hold the syringe steady with respect to his body.

For use, the first support member 16 is first displaced into its retracted position with respect to the second support member and a syringe suitably charged with a required volume of fluid to be injected, is partially positioned within its formations 54 and 56 as defined by the first support member 12. The operating member 64 is next retracted up to a position in which the syringe can be received completely within its formations with the free end of the member 64 effectively abutting the piston rod of the syringe. The operating member 64 is held in this position by means of a pawl and ratchet arrangement 38. In this configuration, with the first support member 12 retracted, and by the suitable adjustment of the abutment formation 70, the free end of the needle of the syringe can be at a position level with or behind the front face 82 of formation 70.

For injecting the fluid in the syringe into the body of a person, the front face 82 of the formation 70 is securely held in a required position against the body of a person in the region where he is to be injected. The arm 31 projecting from the hook formation 30 is then depressed to release the first support member 12 which will clearly cause the needle of the syringe to be inserted into the body of a person as the member 12 approaches the position in which the front end 20 of this element is in the position shown in the drawings. Also, as this position is reached, the rod 34 and thus the operating member 64 is released providing for operation of the piston rod of the syringe and thus the injection of fluid into the body of a person. In this way, the injection of a fluid into the body of a person is completely controlled and since it is possible to keep the device steady with respect to the body of a person by pressing the front face 82 of the abutment formation 70 against the body of the person, very little pain or discomfort will be suffered by a person being injected in this way, even if the person injects himself.

With the spring 18 effectively weaker than the spring 36, for "cocking" the device 10, the operating member 64 need only be retracted which will first cause the first support member 12 to retract and then the rod 34. The formation 84 from which the rod 34 and operating member 64 project can particularly serve to "cock" the device 10 by being pulled in the direction of arrow 86 while holding onto the second support member.

The device 10 as above described may be housed in an upper cover member 88 and a lower cover member 90, the upper member 88 being pivoted to provide access for the location of a syringe.

The device is, in fact, particularly designed to provide for self-injection and, more particularly, for sub-cutaneous injection which is often necessary for sufferers of diabetes who need to inject insulin into their bodies from time to time.  The device of the invention is thus particularly suitable for this purpose.

It will be appreciated that the device of the invention can be formed in various different configurations incorporating the essential principles of the invention which permits the device to be securely held with respect to the body of a person while a needle is inserted into his body and the subsequent injection of a fluid after such insertion.

The Applicant believes that the device of the invention will be particularly suitable for use by diabetes sufferers who require insulin injections from time to time or for similar applications where self-injection is required.  The device is clearly more suitable for sub-cutaneous injections which can be easily attended to by people themselves.

CLAIMS

1.       A device for operating a syringe to inject fluids into the body of a person, which includes

a first support member including locating formations for removably locating and supporting a syringe;

a second support member onto which the first support member is displaceably mounted for displacement with respect to the second support member from a first position into a second position during which the needle of a syringe, supported by the first support member, can be inserted into the body of a person while the second support member is held in a predetermined steady relationship with respect to the body of the person;

an operating member displaceably mounted onto the first support member for displacement with respect to the first support member from a first position into a second position during which it can engage and displace the plunger of a syringe, supported by the first support member, for injecting a fluid from the syringe into the body of a person;

urging means for urging the first support member and the operating member into their second positions;

a first holding mechanism for releasably holding the first support member in its first position, with respect to the second support member, being operable to release the first support member in order to permit its displacement into its second position;    and

a second holding mechanism for releasably holding the operating member in its first position with respect to the first support member being operable to release the operating member in order to permit its displacement into its second position only when the first support member is in its second position with respect to the second support member.

2.      A device as claimed in Claim 1, in which the first support member is linearly displaceable with respect to the second support member between its first position in which the needle of a syringe supported by the first support member in use, is effectively retracted, and its second position in which the needle is inserted into the body of a person.

3.      A device as claimed in Claim 1 or Claim 2, in which the urging means includes two springs which can act respectively on the first support member and the operating member for urging them into their second positions.

4.      A device as claimed in any one of the preceding claims, in which the first holding mechanism is manually operable to release the first support member in order to permit its displacement into its second position.

5.      A device as claimed in any one of the preceding claims in which the first holding mechanism includes a hook formation secured to the second support member and urged into engagement with an engagement formation of the first support member when in its first position, being manually displaceable to release the engagement formation and permit displacement of the first support member into its second position.

6.      A device as claimed in any one of the preceding claims, in which the operating member is linearly displaceable with respect to the first support member between its first position in which the plunger of a syringe supported by the first support member is effectively retracted and its second position in which a fluid contained in the container of the syringe has been ejected by the displacement of the plunger.

7.      A device as claimed in any one of the preceding claims, in which operation of the second holding mechanism is sensitive to the displacement of the first support member and is actuated to release the operating member by means associated with the first support member to permit displacement of the operating member.

8.      A device as claimed in any one of the preceding claims in which the second holding mechanism is adapted to hold the operating member with respect to the first support member in a plurality of positions which determine the first position thereof.

9.     A device as claimed in Claim 8, in which the second holding mechanism is a pawl and ratchet-type mechanism, ratchet formations being defined by the operating member which are engageable by a pawl formation urged into engagement with the ratchet formations to hold the operating member in a required position, the pawl formation being displaceable in response to displacement of the first support member into its second position to release the operating member and permit its displacement into its second position.

10.     A device as claimed in any one of the preceding claims which includes adjustment means for adjusting the distance of insertion of a needle into the body of a person.

11.     A device as claimed in Claim 10, in which the adjustment means is an abutment formation, adjustably securable to the second support member, which abuts the body of a person, in use, and which determines the position of a syringe, when supported by the first support member, with respect to the body of the person and thus the distance of insertion into the body of the person.

12.    A device as claimed in Claim 11, in which the adjustment means includes a pawl and ratchet-type mechanism which permits adjustment of the position of the abutment formation with respect to the second support member.

13.    A device as claimed in any one of the preceding claims in which the locating formations of the first support member can locate syringes having a range of shapes and sizes.

14.    A device as claimed in any one of the preceding claims, in which the operating member defines a piston and the first support member a cylinder within which the piston is linearly displaceable, to provide for the displacement of the operating member.

15.    A device as claimed in Claim 14, in which a fluid outlet means is defined to release fluid from the cylinder at a controlled rate to thereby control the rate of displacement of the operating member.

16.    A device as claimed in Claim 15, in which the fluid outlet means is also in communication with a liquid container defining a closed liquid flow path with the container and the cylinder.

17.    A device as claimed in any one of the preceding claims which includes a cover member mounted onto the second support member being displaceable to permit or inhibit access to a syringe located on the first support member, in use.

18.    The features herein described, or their equivalents, in any patentably novel selection.

FIG. I

FIG. 2

FIG. 3

FIG. 4

0108529

**European Patent Office**

**EUROPEAN SEARCH REPORT**

Application number

EP 83 30 6317

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | FR-A- 953 872 (R. GHIGINI) <br><br> * Whole document * | 1-11, 13,14 | A 61 M 5/20 |
| Y | | 12 | |
| Y | GB-A-1 133 555 (E. VALENTIN) <br> * Figure 5; page 2, lines 1-79 * | 12 | |
| X | US-A-2 295 849 (G.L. KAYDEN) <br><br> * Whole document * | 1,2,4-11,14,15 | |
| Y | | 16 | **TECHNICAL FIELDS SEARCHED (Int. Cl. 3)** |
| Y | US-A-4 067 334 (J.G. HALLER) <br> * Column 4, lines 27-49; figure 1 * | 16 | A 61 M |
| X | GB-A- 909 898 (D.T. LEWIS) <br><br> * Whole document * | 1-7,10,13,17 | |
| X | FR-A-1 014 881 (F. HANON) <br><br> * Page 2, lines 20-39; figure 2 * | 1-9,13,17 | |
| | --- -/- | | |

The present search report has been drawn up for all claims

| Place of search <br> THE HAGUE | Date of completion of the search <br> 17-01-1984 | Examiner <br> WOLF C.H.S. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl. 3) |
|---|---|---|---|
| X | FR-A-1 100 079 (R. ANAVE) <br><br> * Whole document * | 1-9,11 ,13,17 | |
| X | EP-A-0 004 365 (A. NATTERMANN & CIE.) <br><br> * Abstract * | 1,2,4-6,10, 12 | |
| A | | 3,8,9 | |
| A | FR-A- 392 105 (M. LOMBARDO) <br> * Figures 3,4 * | 14 | |
| A | DE-C- 562 328 (E. KOCH) <br> * Whole document * | 14 | |
| | | | TECHNICAL FIELDS SEARCHED (Int. Cl. 3) |

The present search report has been drawn up for all claims

| Place of search <br> THE HAGUE | Date of completion of the search <br> 17-01-1984 | Examiner <br> WOLF C.H.S. |
|---|---|---|